# EUROPEAN PATENT APPLICATION

(11) **EP 1 634 947 A1**
(43) Date of publication of application: **15.03.2006**
(21) Application number: 04745964.9
(22) Date of filing: 09.06.2004
(51) Int. Cl.: C12N 1/06, C12N 5/06, C12N 15/09, C12P 21/02

(54) **EXTRACT FROM CULTURED MAMMALIAN CELL, PROCESS FOR PREPARATION THEREOF AND METHOD OF CELL-FREE PROTEIN SYNTHESIS USING THE EXTRACT**

(30) Priority: 10.06.2003 JP 2003165390
(71) Applicant: SHIMADZU CORPORATION, Kyoto-shi, Kyoto 604-8511 (JP)
(72) Inventor: SUZUKI, Takashi, c/o Shimadzu Corporation, Kyoto-shi, Kyoto 604-8511 (JP); EZURE, Toru, c/o Shimadzu Corporation, Kyoto-shi, Kyoto 604-8511 (JP); ITO, Masaaki, c/o Shimadzu Corporation, Kyoto-shi, Kyoto 604-8511 (JP); HIGASHIDE, Shoken, c/o Rengo Co., Ltd., Osaka-shi, Osaka 553-0007 (JP); ENDO, Koki, Yokohama-shi, Kanagawa 236-0042 (JP)
(74) Representative: Kilian, Helmut
(86) International application number: PCT/JP2004/008422
(87) International publication number: WO 2004/111203

(57) **Abstract**

A method for preparing a cultured mammalian cell extract liquid, comprising at least the step of rapidly freezing a cultured mammalian cell suspended in a solution for extraction; a culturedmammalian cell extract liquid prepared by the process; and a method for cell-free protein synthesis using the extract liquid. Preferably, the method for cell-free protein synthesis comprises the step of prior to conducting of synthetic reaction, effecting incubation of a reaction liquid for cell-free protein synthesis in a state of containing components other than exogenous mRNA for a specified period of time.

SEQUENCE LISTING

<110> SHIMADZU CORPORATION

<120> culutured mammalian cell extract, and method for preparity
same, method for producing proteins using same

<130> G104052 WO

<150> JP 2003-165390
<151> 2002-6-10

<160> 3

<210> 1
<211> 7
<212> DNA
<213> Artificial Sequence

<220>
<223> Luc T7-F3-Kpn

<400> 1
ggggtaccat ggaagacgcc aaaaacataa 30

<210> 2
<211> 29
<212> DNA
<213> Artficial Sequence

<220>
<223> Luc T7-R4-Kpn

<400> 2
ggggtacctt acaatttgga ctttccgcc 29

<210> 3
<211> 20
<212> DNA
<213> Artficial Sequence

<220>
<223> T7 promoter

<400> 3
taatacgact cactataggc 20

## Description

### TECHNICAL FIELD

The present invention relates to a method for cell-free protein synthesis using an extract liquid from cultured mammalian cell, and a process for the preparation of said extract liquid.

### BACKGROUND ART

In recent years, genetic information of many organisms, such as human genome, has been decoded. Under the circumstances, functional analysis of proteins and creation of genomic medicine based on such genetic information have been attracting attention for postgenomic studies. Application and utilization of proteins corresponding to such genetic information for pharmaceutical products and the like requires easy synthesis of extensive kinds of proteins in a short time.

At present, expression systems using viable cells (hereinafter sometimes to be referred to as "cell-system") of yeast, cultured mammalian cell and the like by the gene recombination technique have been widely utilized as the production methods of proteins. However, viable cells show a propensity toward elimination of exogenous proteins for their functional retention, and there are many proteins that cannot be expressed easily since expression of cytotoxic proteins in viable cells prevents cell growth.

On the other hand, as a production method of protein free of a cell-system, cell-free protein synthesis has been known, which includes adding a substrate, an enzyme and the like to a cell rupture, extract liquid and the like to provide a wide choice of genetic information translation systems of organisms in test tubes, and reconstructing a synthetic system capable of linking the necessary number of amino acid residues in a desired order using an mRNA encoding an object protein. Such a cell-free protein synthesis is relatively free of the limitation imposed on the above-mentioned cell-system protein synthesis, and is capable of synthesizing proteins without killing the organism. In addition, because the production of protein does not accompany operations of culture and the like, the protein can be synthesized in a short time as compared to cell-systems. Moreover, inasmuch as the cell-free protein synthesis also affords a large scale production of proteins consisting of amino acid sequences not utilized by the organism, it is expected to be a promising expression method. As a cell rupture or extract liquid to be applied to the cell-free protein synthesis, use of various substances of biological derivation has been considered and investigations are underway.

Although there are conventionally-known various extract liquids for cell-free protein synthesis, it can be considered that an extract liquid derived from a cultured mammalian cell more closely related to a human cell is desirable in view of posttranslational modification such as glycosylation because such an extract liquid allows human protein to be expressed with full activity being retained. At present, as extract liquids for cell-free protein synthesis derived from cultured mammalian cells, there have been reported a rabbit reticulocyte system (see, for example, Non-Patent Document 1 : Pelham et al. , Eur. J. Biochem. 67, 247 - 256 (1976)) and a Chinese hamster ovary (CHO) system (see, for example, Patent Document 1: JP-A-2000-325076).

In the case of a rabbit reticulocyte system, however, lysate is prepared using blood collected from the ear of a rabbit after the lapse of 4 to 5 days from subcutaneous injection of an acetylphenylhydrazine solution into the rabbit. In this way, a rabbit reticulocyte system involves very complicated manipulations. On the other hand, in the case of a CHO system, an extract liquid is prepared by applying pressure to CHO cells in an inert gas atmosphere and, then, reducing the pressure, thereby to rupture the CHO cells. In this case, special devices and tools are necessary for preparing the extract liquid. Moreover, complicated manipulations are necessary for the setting of the conditions, because protein synthesis ability of the cell-free system vastly change depending on the number of cells, gas pressure and pressurization time during the preparation of the extract liquid. In addition, the amount of protein synthesized using the extract liquid obtained by this method is extremely small, which is of the level that the activity of the protein synthesis can be only measured by the uptake of the radiolabeled amino acid.

Therefore, the development of a cultured mammalian cell -derived extract liquid, which is easy to prepare and which affords synthesis of a large amount of protein, and the preparation method thereof is desired.

### DISCLOSURE OF THE INVENTION

### Objects of the Invention

The present invention has been made to solve the above-mentioned problems and aims at providing a preparation method of a cultured mammalian cell extract liquid for cell-free protein synthesis, which liquid is easy to prepare and affords synthesis of a protein in a higher amount than by conventional liquids, the cultured mammalian cell extract liquid, and a cell-free protein synthesis method using the culturedmammalian cell extract liquid.

As a result of the intensive studies conducted by the present inventors in an attempt to solve the above-mentioned problems, the present invention has been completed. Accordingly, the present invention provides the following.
(1) A method for preparing a cultured mammalian cell extract liquid, comprising at least the step of rapidly freezing a cultured mammalian cell suspended in a solution for extraction.
(2) The preparation method according to claim 1 , further comprising the step of thawing the cultured mammalian cell after rapid freezing, and subjecting the cell to centrifugation.
(3) The preparation method according to claim 1 or 2, wherein the cultured mammalian cell is rapidly frozen with liquid nitrogen.
(4) The preparation method according to claim 2 or 3, wherein the cultured mammalian cell rapidly frozen is thawed in a water bath or ice-water bath at -10°C - 20°C.
(5) The preparation method according to any one of claims 1 to 4, wherein the solution for extraction contains a protease inhibitor.
(6) The preparation method according to any one of claims 1 to 5, wherein the cultured mammalian cell is a cultured cell derived from lymphoma.
(7) The preparation method according to any one of claims 1 to 5, wherein the cultured mammalian cell is a cultured cell derived from a gonad.
(8) The preparation method according to claim 7, wherein the cultured mammalian cell is a cultured cell derived from Chinese hamster ovary (CHO).
(9) The preparation method according to claim 8, wherein the Chinese hamster ovary (CHO) is derived from CHO K1-SFM.
(10) A cultured mammalian cell extract liquid, which is prepared by the method according to any one of claims 1 to 9.
(11) A method for cell-free protein synthesis, comprising use of the cultured mammalian cell extract liquid according to claim 10.
(12) The method for cell-free protein synthesis according to claim 11, comprising the steps of;
   effecting incubation of a reaction liquid for cell-free protein synthesis containing components other than an exogenous mRNA, and then
   adding an exogenous mRNA into the reaction liquid to conduct synthetic reaction.
(13) The method for cell-free protein synthesis according to claim 12, wherein the incubation is carried out at 0°C to 50°C.

### BRIEF DESCRIPTION OF THE DRAWINGS

Fig. 1 is a graph of experimental results of Example 2, which shows the influence of presence or absence of an incubation step (incubation of a cell-free protein synthesis reaction liquid in a state of containing components other than exogenous mRNA for a specified period of time prior to conducting synthetic reaction) on an amount of synthesized luciferase.

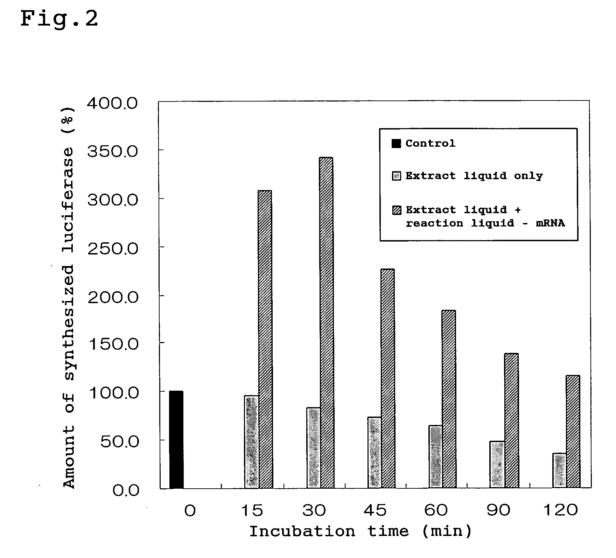

Fig. 2 is a graph of experimental results of Example 2, which shows the influence of the composition of a reaction liquid and a retention time in the incubation step on the amount of synthesized luciferase.

### MODES FOR CARRYING OUT THE INVENTION

The present invention provides a method for preparing a cultured mammalian cell extract liquid by the extraction from a cultured mammalian cell, which is largely characterized in comprising at least a step of rapidly freezing a cultured mammalian cell suspended in a solution for extraction. By preparing the cultured mammalian cell extract liquid by such a method, the cells can be ruptured under milder conditions as compared to the above-mentioned conventional method described in patent document 1, and the components essential for cell-free protein synthesis can be taken out from the cell without damage, which in turn affords easy preparation of a cultured mammalian cell extract liquid having higher capacity to synthesize a protein in a cell-free system than an extract liquid produced by a conventional method. According to the method of the present invention, moreover, contamination with RNase and the like from the tools and the like can be prevented, and incorporation of a substance inhibiting translation reaction, which is of concern in the case of cell rupture methods using a reagent such as detergent and the like, can be avoided.

The preparation method of the present invention requires rapid freezing of cultured mammalian cells suspended in a solution for extraction. In the preparation method of the present invention, by the "rapid freezing" is meant that a cultured mammalian cell is frozen in not longer than 10 sec, preferably not longer than 2 sec. When the cultured mammalian cell is not rapidly frozen in the present invention, the components essential for protein synthesis may be inactivated, and the above-mentioned effect of the present invention may not be achieved.

As mentioned above, the cultured mammalian cell is rapidly frozen at a temperature of generally not higher than -80°C, preferably not higher than -150°C. This is because the protein synthesis ability tends to be degraded when the cell is rapidly frozen at a temperature exceeding -80°C, since the components essential for the protein synthesis are inactivated.

The above-mentioned rapid freezing of the cultured mammalian cell can be realized by, for example, using an inert gas such as liquid nitrogen, liquid helium and the like, and the like. It is preferable to use liquid nitrogen because it is easily available and economical.

According to the preparation method of the present invention, as long as at least a step for rapidly freezing a cultured mammalian cell suspended in a solution for extraction is included in the extraction from a cultured mammalian cell, other steps are free of any particular limitation. For example, cultured mammalian cells may be ruptured and extracted by various methods conventionally employed for obtaining an extract liquid for cell-free protein synthesis from *Escherichia coli,* wheat germ and the like, such as a method comprising mashing the cells in a mortar with a pestle, a method using a dounce homogenizer, a method using glass beads and the like. Because cultured mammalian cells are easily ruptured as compared to an extract liquid for cell-free protein synthesis obtained from *Escherichia coli,* wheat germ and the like, and because an extract liquid having high protein synthesis ability can be obtained since the rupture method including freeze-thawing alone affords an extract liquid containing the components essential for protein synthesis in a state of retaining the activity, it is preferable to rupture the above-mentioned cultured mammalian cell by rapidly freezing, thawing and centrifuging the cultured mammalian cell.

When the above-mentioned rapidly frozen cultured mammalian cell is to be thawed and centrifuged, the cell is thawed in a water bath or ice-water bath at -10°C to 20°C, by leaving the cell to stand at room temperature (25°C) and the like. To prevent inactivation of the components essential for protein synthesis and to certainly prevent degradation of protein synthesis ability, the cell is preferably thawed in a water bath or ice-water bath at 0°C to 20°C (particularly 4°C to 10°C).

The thawed cultured mammalian cell is centrifuged under the conditions generally employed in the pertinent field (10,000×g - 50,000×g, 0°C - 10°C, 10 min - 60 min).

The cultured mammalian cell to be used in the present invention is not subject to any particular limitation, and, for example, cultured cells derived from conventionally-known mammalian such as human, rat, mouse, monkey can be appropriately used.

Furthermore, the cultured mammalian cell in the present invention may be a cell derived from any tissue, and, for example, blood cell, gonad-derived cell, lymphoma-derived cell, other tumor cell, stem cell and the like can be used without any particular limitation. Of these, lymphoma-derived cells are preferably used because suspension culture is possible and therefore they are easily cultured and subcultured. In the case of CHO cells, not only suspension culture but also culture using a serum-free medium are possible; therefore, CHO cells are more easily cultured and subcultured. Further, such CHO cells are widely used in a cell-system and have high protein synthesis ability. From the fact, it can be expected that the same will be true in a cell-free system. For this reason, CHO cells are preferably used.

In the present invention, the cell is not limited to a cultured mammalian cell derived from a single tissue of a single species of mammal, and it may be extracted from a cultured mammalian cell derived from plural kinds of tissues of a single species of mammal, a cultured mammalian cell derived from a single tissue of plural species of mammals, or a cultured mammalian cell derived from plural kinds of tissues of plural species of mammals.

A solution for extraction to be used for the preparation method of the present invention is not particularly limited, but it preferably contains at least a protease inhibitor. When a solution for extraction containing a protease inhibitor is used, the protease activity contained in an extract derived from the cultured mammalian cell is inhibited, thereby preventing undesired decomposition of the active protein in the extract due to protease, which in turn effectively draws out advantageously the protein synthesis ability that the extract derived from the cultured mammalian cell has.

The above-mentioned protease inhibitor is not particularly limited as long as it can inhibit the activity of protease, and, for example, phenylmethanesulfonyl fluoride (hereinafter sometimes to be referred to as "PMSF"), aprotinin, bestatin, leupeptin, pepstatin A, E-64 (L-trans-epoxysuccinyl-L-leucylamido(4-guanidino)butane), ethylenediaminetetraacetic acid, phosphoramidon and the like can be used. Since an extract liquid derived from a cultured mammalian cell contains serine protease, the use of PMSF, which works as an inhibitor having high specificity to serine protease, is preferable among those mentioned above. It is possible to use not only one kind of protease inhibitor but also a mixture (protease inhibitor cocktail) of several kinds of protease inhibitors.

The content of the protease inhibitor in the solution for extraction is free of any particular limitation, but it is preferably 1 µM - 50 mM, more preferably 0.01 mM - 5 mM, because decomposition of the enzyme necessary for the action of the present invention can be preferably inhibited. This is because the decomposition activity of protease often cannot be suppressed sufficiently when the protease inhibitor content is less than 1 µM, and the protein synthesis reaction tends to be inhibited when the protease inhibitor content exceeds 50 mM.

The solution for extraction to be used for the present invention preferably contains, in addition to the above-mentioned protease inhibitor, at least a potassium salt, a magnesium salt, dithiothreitol and a buffer.

The above-mentioned potassium salt is free of any particular limitation as long as it does not inhibit the action of the present invention, and can be used in a general form, such as potassium acetate, potassium carbonate, potassium hydrogen carbonate, potassium chloride, dipotassium hydrogen phosphate, dipotassium hydrogen citrate, potassium sulfate, potassium dihydrogen phosphate, potassium iodide, potassium phthalate and the like, with preference given to potassium acetate. Potassium salt acts as a cofactor in the protein synthesis reaction.

The content of the potassium salt in the solution for extraction is free of any particular limitation, but from the aspect of preservation stability, it is preferably 10 mM - 500 mM, more preferably 20 mM - 300 mM, in the case of a monovalent potassium salt, such as potassium acetate and the like. When the content of the potassium salt is less than 10 mM or more than 500 mM, the components essential for protein synthesis tend to become unstable.

The above-mentioned magnesium salt is free of any particular limitation as long as it does not inhibit the action of the present invention, and can be used in a general form such as magnesium acetate, magnesium sulfate, magnesium chloride, magnesium citrate, magnesium hydrogen phosphate, magnesium iodide, magnesium lactate, magnesium nitrate, magnesium oxalate and the like, with preference given to magnesium acetate. Magnesium salt also acts as a cofactor in the protein synthesis reaction.

The content of the magnesium salt in the solution for extraction is free of any particular limitation, but from the aspect of preservation stability, it is preferably 0.1 mM - 10 mM, more preferably 0.5 mM - 5 mM, in the case of a divalent salt, such as magnesium acetate and the like. When the content of the magnesium salt is less than 0.1 mM or more than 10 mM, the components essential for protein synthesis tend to become unstable.

The above-mentioned dithiothreitol (hereinafter sometimes to be referred to as "DTT") is added for prevention of oxidization, and is preferably contained in an amount of 0.1 mM - 10 mM, more preferably 0.5 mM - 5 mM, in the solution for extraction. When the content of DTT is less than 0.1 mM or more than 10 mM, the components essential for protein synthesis tend to become unstable.

The above-mentioned buffer imparts a buffer capacity, and is, in an extract liquid (= a solution for extraction + extract) prepared by extraction using a solution for extraction, added for prevention of denaturation of an extract caused by a radical change in pH, which is due to, for example, addition of an acidic or basic substance and the like. Such buffer is free of any particular limitation, and, for example, HEPES-KOH, Tris-HCl, acetic acid-sodium acetate, citric acid-sodium citrate, phosphoric acid, boric acid, MES, PIPES and the like can be used.

The buffer is preferably one that maintains the pH of the extract liquid obtained at 4 - 10, more preferably pH 6 - 8.5. When the pH of the extract liquid is less than 4 or more than 10, the components essential for the reaction of the present invention may be denatured. From this aspect, the use of HEPES-KOH (pH 6 - 8.5) is particularly preferable among the above-mentioned buffers.

While the content of the buffer in the solution for extraction is free of any particular limitation, it is preferably 5 mM - 200 mM, more preferably 10 mM - 100 mM, to maintain preferable buffer capacity. When the content of the buffer is less than 5 mM, pH tends to change radically due to the addition of an acidic or basic substance, which in turn may cause denaturation of the extract in the extract liquid prepared using less than 5 mM of the buffer, and when the content of the buffer exceeds 200 mM, the salt concentration becomes too high and the components essential for protein synthesis tend to become unstable.

Further, it is preferred that calcium salt and glycerol are further added to the solution for extraction to obtain an extract liquid having improved protein synthesis ability. The calcium salt is free of any particular limitation as long as it does not inhibit the action of the present invention, and can be used in a general form such as calcium chloride, calcium acetate, calcium sulfate, calcium citrate, calcium iodide, calcium lactate, calciumnitrate, calciumoxalate, and the like. In particular, calcium chloride is preferably used. In this case, the content of calcium chloride is not particularly limited. For effective exertion of the effect of the above-mentioned improved protein synthesis ability, it is preferably contained in the range of 0.1 mM - 10 mM, more preferably 0.5 mM - 5 mM. In addition, while the amount of glycerol to be added is not particularly limited, for effective exertion of the effect of the above-mentioned improved protein synthesis ability, it is preferably added in a proportion of 5 (v/v)% - 80 (v/v)%, more preferably 10 (v/v)% - 50 (v/v)%.

In the preparation method of the cultured mammalian cell extract liquid of the present invention, the steps after cell rupture to the obtainment of a cultured mammalian cell extract liquid for cell-free protein synthesis are not particularly limited.

For example, in a case where cultured mammalian cells suspended in the solution for extraction are rapidly frozen and, then, thawed and centrifuged, the resultant supernatant (supernatant 1) after the centrifugation may be used as it is as a cultured mammalian cell extract liquid. The supernatant 1 may be further subjected to centrifugation to use the resultant supernatant (supernatant 2) as a cultured mammalian cell extract liquid. The supernatant 1 can be subjected to centrifugation under the same conditions as described above (that is, 10,000×g - 50,000×g, 0°C - 10°C, 10 min - 60 min).

Further, the supernatant 1 or supernatant 2 prepared as described above may be subjected to gel filtration to prepare filtrate. In this case, fractions having the highest absorbance and its vicinity at 280 nm are separated from the filtrate, and may be used as an extract liquid. However, from the viewpoint of protein synthesis efficiency, it is preferred that an extract liquid is prepared without such gel filtration and separation of fractions.

In a case where the supernatant 1 or supernatant 2 is subjected to gel filtration to obtain filtrate, and then fractions having the highest absorbance and its vicinity at 280 nm are separated from the filtrate, the following procedures are concretely carried out.

For example, a desalting column PD-10 (manufactured by Amersham Biosciences) is used. According to conventional methods, the column is equilibrated with a buffer solution for gel filtration, a sample is fed and eluted using the above-mentioned solution for extraction. As the above-mentioned buffer solution for gel filtration, conventionally known buffer solutions having appropriate compositions can be used without any particular limitation. For example, a buffer solution for gel filtration containing 10 mM - 100 mM HEPES-KOH (pH 6 - 8.5), 20 mM - 300 mM potassium acetate, 0.5 mM - 5 mM magnesium acetate, 0.5 mM - 5 mM DTT and 0.01 mM - 5 mM PMSF can be used. The filtrate obtained by gel filtration may be fractionated into 0.1 mL - 1 mL fractions as in general gel filtration, and 0.4 mL - 0.6 mL is preferably used as one fraction, for efficiently obtaining a fraction having high protein synthesis ability.

Next, a fraction having an absorbance at 280 nm of not less than 10 is separated from the filtrate after gel filtration. According to the treatment, an absorbance at 280 nm is measured about each fractions, fractions having the highest absorbance and its vicinity at 280 nm are separated, using instruments such as Ultrospec 3300 pro (manufactured by Amersham Biosciences), to give the extract liquid.

The cultured mammalian cell to be subjected to the preparation method of the present invention is preferably washed in advance with a wash solution, before the above-mentioned rapid freezing, so that the medium used for culture will not be brought into the translation reaction. The composition of a wash solution may be the same composition as the aforementioned solution for extraction. Washing with a wash solution include addition of the wash solution to a cultured mammalian cell, and centrifugation thereof (e.g., in the condition of 700×g, 10 min, 4°C).

The amount of the wash solution to be used for the washing is preferably 5 mL - 100 mL, more preferably 10 mL - 50 mL, per 1 g (wet weight) of cultured mammalian cell, for complete removal of the medium.

The frequency of washing is preferably 1-5 times, more preferably 2-4 times.

In addition, while the amount of the cultured mammalian cell to be subjected to the preparation method of the present invention is not particularly limited, it is preferably 0.1 g - 5 g, more preferably 0.5 g - 2 g, per 1 mL of the solution for extraction, to maintain optimum extraction efficiency.

The cultured mammalian cell extract liquid prepared according to the method of the present invention can be preferably used for cell-free protein synthesis. The cultured mammalian cell extract liquid preferably contains an extract derived from the cultured mammalian cell in a protein concentration of 1 mg/mL - 200 mg/mL, more preferably 10 mg/mL - 100 mg/mL. When the content of the extract in a protein concentration is less than 1 mg/mL, the concentration of the components essential for achieving the present invention becomes lower, risking completion of sufficient synthesis reaction. When the content of the extract in a protein concentration exceeds 200 mg/mL, the extract liquid itself comes to have high viscosity, making operation difficult.

The content of the extract derived from a cultured mammalian cell in the extract liquid is determined by measuring the protein concentration. For example, it is determined by measuring the protein concentration using a BCA Protein assay Kit (manufactured by PIERCE). To be specific, 0.1 mL of a sample is added to 2 mL of a reaction reagent, the mixture is reacted at 37°C for 30 min and, using a spectrophotometer (Ultrospec 3300 pro, manufactured by Amersham Biosciences), the absorbance at 562 nm is measured. For control, bovine serum albumin (BSA) is generally used to form a standard curve, whereby the measurement is completed.

Whether or not the extract contained in the extract liquid is derived from a cultured mammalian cell is determined by, for example, base sequence analysis of ribosomal RNA in an extract liquid.

The extract liquid of the present invention is preferably realized to contain the extract derived from a culturedmammalian cell in a protein concentration of 10 mg/mL - 100 mg/mL, concurrently with 20 mM - 300 mM of potassium acetate, 0.5 mM - 5 mM of magnesium acetate, 0.5 mM - 5 mM of DTT, 0.01 mM - 5 mM of PMSF and 10 mM - 100 mM of HEPES-KOH (pH 6 - 8.5). It is preferred that the extract liquid further contains 0.5 mM - 5 mM of calcium chloride salt and 10 (v/v)% - 50 (v/v)% of glycerol in addition to the above-mentioned components.

The present invention also provides a method for cell-free protein synthesis using the above-described extract liquid. By carrying out protein synthesis reaction using an extract liquid obtained by the method according to the present invention, it is possible to synthesize any protein, for example, proteins cytotoxic to living cells, in a short time. Further, since such an extract liquid uses an extract derived from a cultured cell of a mammal which is eukaryote, it is also possible to synthesize glycoprotein in a cell-free system. In this way, various proteins can be synthesized without any limitation. It is to be noted that in the present invention cell-free protein synthesis reaction refers to protein synthesis reaction in only a cell-free translation system in which a protein is synthesized by decoding of the information of mRNA.

The composition of a reaction liquid for cell-free protein synthesis which is prepared using an extract liquid obtained by the method according to the present invention is not particularly limited, and conventionally known composition can be appropriately selected. However, the reaction liquid is preferably prepared in such a manner that the extract liquid described above is contained in a proportion of 10 (v/v)% - 80 (v/v)%, particularly 30 (v/v)% - 60 (v/v)%. In other words, it is preferably prepared in such a manner that the content of the extract derived from cultured mammalian cells is 0.1 mg/mL - 160 mg/mL, more preferably 3 mg/mL - 60 mg/mL, throughout the above-mentioned reaction liquid. When the content of the extract is less than 0.1 mg/mL or above 160 mg/mL in a protein concentration, the synthesis rate of the object protein may become lower.

The reaction liquid for cell-free protein synthesis preferably contains, as components other than an extract liquid obtained by the method according to the present invention, at least exogeneous mRNA, potassium salt, magnesium salt, DTT, adenosine triphosphate, guanosine triphosphate, creatine phosphate, creatine kinase, amino acid component, RNase inhibitor, tRNA, and buffer. By using such a reaction liquid for translation reaction, it is possible to synthesize a large amount of protein in a short time.

The exogenous mRNA used for the above-mentioned reaction liquid means an mRNA which is not derived from the cultured mammalian cell used for preparation of extract liquid. As regards the exogenous mRNA, a protein (including peptide) to be encoded thereby is not particularly limited as long as the mRNA is not derived from a cultured mammalian cell, and the mRNA may encode a toxic protein or a glycoprotein. Whether the mRNA contained in the reaction liquid is an exogenous mRNA or mRNA derived from a cultured mammalian cell used for preparation of extract liquid can be determined by isolating and purifying the mRNA from a reaction liquid, synthesizing cDNA using a reverse transcriptase, analyzing a base sequence of the obtained cDNA and comparing the base sequences with the base sequences of known exogenous mRNAs.

The exogenous mRNA to be used for the reaction liquid is not particularly limited as regards the number of bases and all the mRNAs may not have the same number of bases as long as they can synthesize the object protein. In addition, as long as the sequences are homologous to the degree allowing synthesis of the object protein, plural bases of each mRNA may be deleted, substituted, inserted or added.

The exogenous mRNA to be used for the present invention may be a commercially available one or an mRNA obtained by inserting ORF (Open reading frame) of the object protein into the downstream of the 5'-β-globin leader sequence of a commerciallyavailablevector, such as pT_{N}TVector (manufactured by Promega), and performing a transcription reaction using the resulting vector. Furthermore, an exogenous mRNA having a cap structure resulting from the addition of methylated ribonucleotide and the like during transcription reaction may be used.

The exogenous mRNA is preferably contained in the reaction liquid in a proportion of 1 µg/mL - 1000 µg/mL, more preferably 10 µg/mL - 500 µg/mL, in view of the protein synthesis rate. When the amount of exogenous mRNA is less than 1 µg/mL or exceeds 1000 µg/mL, the rate of protein synthesis tends to decrease.

As the potassium salt in the reaction liquid, various potassium salts described above as a component of solution for extraction, preferably potassium acetate, can be preferably used. The potassium salt is preferably contained in the reaction liquid in a proportion of 10 mM - 500 mM, more preferably 20 mM - 300 mM, from the same aspect of the potassium salt in the aforementioned solution for extraction.

As the magnesium salt in the reaction liquid, various magnesium salt described above as a component of solution for extraction, preferably magnesium acetate, can be preferably used. The magnesium salt is preferably contained in the reaction liquid in a proportion of 0.1 mM - 10 mM, more preferably 0.5 mM - 5 mM, from the same aspect of the magnesium salt in the aforementioned extract liquid.

DTT is preferably contained in the reaction liquid in a proportion of 0.1 mM - 10 mM, more preferably 0.5 mM - 5 mM, from the same aspect of DTT in the aforementioned solution for extraction.

The adenosine triphosphate (hereinafter sometimes to be referred to as "ATP") in the reaction liquid is preferably contained in the reaction liquid in a proportion of 0.01 mM - 10 mM, more preferably 0.1 mM - 5 mM, in view of the rate of protein synthesis. When ATP is contained in a proportion of less than 0.01 mM or above 10 mM, the synthesis rate of the protein tends to become lower.

The guanosine triphosphate (hereinafter sometimes to be referred to as "GTP") in the reaction liquid preferably contained in the reaction liquid in a proportion of 0.01 mM - 10 mM, more preferably 0.05 mM - 5 mM, in view of the rate of protein synthesis. When GTP is contained in a proportion of less than 0.01 mM or above 10 mM, the synthesis rate of the protein tends to become lower.

The creatine phosphate in the reaction liquid is a component for continuous synthesis of protein and added for regeneration of ATP and GTP. The creatine phosphate is preferably contained in the reaction liquid in a proportion of 1 mM - 200 mM, more preferably 10 mM - 100 mM, in view of the rate of protein synthesis. When creatine phosphate is contained in a proportion of less than 1 mM, sufficient amounts of ATP and GTP may not be regenerated easily. As a result, the rate of protein synthesis tends to become lower. When the creatine phosphate content exceeds 200 mM, it acts as an inhibitory substance and the rate of protein synthesis tends to become lower.

The creatine kinase in the reaction liquid is a component for continuous synthesis of protein and added along with creatine phosphate for regeneration of ATP and GTP. The creatine kinase is preferably contained in the reaction liquid in a proportion of 1 µg/mL - 1000 µg/mL, more preferably 10 µg/mL - 500 µg/mL, in view of the rate of protein synthesis. When the creatine kinase content is less than 1 µg/mL, sufficient amount of ATP and GTP may not be regenerated easily. As a result, the rate of protein synthesis tends to become lower. When the creatine kinase content exceeds 1000 µg/mL, it acts as an inhibitory substance and the synthesis rate of the protein tends to become lower.

The amino acid component in the reaction liquid contains at least 20 kinds of amino acids, i.e., valine, methionine, glutamic acid, alanine, leuicine, phenylalanine, glycine, proline, isoleucine, tryptophan, asparagine, serine, threonine, histidine, aspartic acid, tyrosine, lysine, glutamine, cystine and arginine. This amino acid includes radioisotope-labeled amino acid. Where necessary, modified amino acid may be contained. The amino acid component generally contains almost the same amount of various kinds of amino acids.

In the present invention, the above-mentioned amino acid component is preferably contained in the reaction liquid in a proportion of 1 µM - 1000 µM, more preferably 10 µM - 200 µM, in view of the rate of protein synthesis. When the amount of the amino acid component is less than 1 µM or above 1000 µM, the synthesis rate of the protein tends to become lower.

The RNase inhibitor in the reaction liquid is added to prevent RNase, which is derived from cultured mammalian cells contaminating the extract liquid, from undesirably digesting exogenous mRNA and tRNA, thereby preventing synthesis of protein, during cell-free protein synthesis of the present invention. It is preferably contained in the reaction liquid in a proportion of 0.1 U/µL - 100 U/µL, more preferably 0.5 U/µL - 10 U/µL. When the amount of RNase inhibitor is less than 0.1 U/µL, the degradation activity of RNase often cannot be suppressed sufficiently, and when the amount of the RNase inhibitor exceeds 100 U/µL, protein synthesis reaction tends to be inhibited.

The tRNA in the reaction liquid contains almost the same amount of each of the tRNAs corresponding to the above-mentioned 20 kinds of amino acids. In the present invention, tRNA is preferably contained in the reaction liquid in a proportion of 1 µg/mL - 1000 µg/mL, more preferably 10 µg/mL - 500 µg/mL, in view of the rate of protein synthesis. When the amount of tRNA is less than 1 µg/mL or exceeds 1000 µg/mL, the rate of protein synthesis tends to become lower.

The buffer to be contained in the reaction liquid is preferably similar to those used for the aforementioned extract liquid of the present invention, and the use of HEPES-KOH (pH 6 - 8.5) is preferable for the same reasons. The buffer is preferably contained in the amount of 5 mM - 200 mM, more preferably 10 mM - 100 mM, from the same aspect as in the aforementioned buffer contained in the extract liquid.

It is preferred that the reaction liquid further contains spermidine for the purpose of enhancing the rate of translation reaction. Spermidine is preferably contained in the reaction liquid in a proportion of 0.01 mM - 10 mM, more preferably 0.05 mM - 5 mM. When the amount of spermidine is less than 0.01 mM, the ability to promote translation tends to be inadequate. On the other hand, when the amount of spermidine exceeds 10 mM, protein synthesis reaction tends to be inhibited.

It is also preferred that the reaction liquid further contains calcium salt. Examples of calcium salt to be added include the various calcium slats mentioned above as a component of the solution for extraction. In particular, calcium chloride can be preferably used. The calcium salt is preferably contained in the reaction liquid in a proportion of 0.05 mM - 10 mM, more preferably 0.1 mM - 5 mM, from the same viewpoint of the calcium salt in the above-described solution for extraction.

In other words, the reaction liquid using the extract liquid obtained by the method of the present invention is preferably made to contain the extract liquid in a proportion of 30 (v/v)% - 60 (v/v)%, together with 20 mM - 300 mM of potassium acetate, 0.5 mM - 5 mM of magnesium acetate, 0.5 mM - 5 mM of DTT, 0.1 mM - 5 mM of ATP, 0.05 mM - 5 mM of GTP, 10 mM - 100 mM of creatine phosphate, 10 µg/mL - 500 µg/mL of creatine kinase, 10 µM - 200 µM of amino acid component, 0.5 U/µL - 10 U/µL of RNase inhibitor, 10 µg/mL - 500 µg/mL of tRNA, 10 µg/mL - 500 µg/mL of exogenous mRNA and 10 mM - 100 mM of HEPES-KOH (pH 6 - 8.5). In addition, the reaction liquid is more preferably made to contain 0.05 mM - 5 mM of spermidine and 0.1 mM - 5 mM of calcium chloride in addition to the above.

The cell-free protein synthesis reaction using the above-mentioned reaction liquid is performed in, for example, a conventionally known low temperature incubator. The reaction temperature is generally within the range of 10°C - 40°C, preferably 20°C -30°C. When the reaction temperature is lower than 10°C, the synthesis rate of the protein tends to become lower, and when the reaction temperature exceeds 40°C, the essential components tend to be denatured. The reaction time is generally 1 hr - 72 hr, preferably 3 hr - 24 hr.

Further, prior to conducting such reaction, incubation is preferably carried out for a specified period of time in a state where the components of the reaction liquid composition other than mRNA are added to the extract liquid. Such incubation is carried out in, for example, a conventionally known low-temperature incubator. The incubation time is usually in a range of 0°C - 50°C, preferably 15°C - 37°C. When the incubation temperature is less than 0°C, it is difficult to achieve the incubation effect. On the other hand, when the incubation temperature exceeds 50°C, essential components tend to be denatured. The incubation time is usually 1 min - 120 min, preferably 10 min - 60 min.

The protein synthesized by using the above-mentioned reaction liquid is free of any particular limitation. The amount of protein synthesized can be measured by activity assay of enzyme, SDS-PAGE, immunoassay and the like.

The protein synthesized by the cell-free protein synthesis method of the present invention is free of any particular limitation.

### EXAMPLES

The present invention will be described more specifically by way of the following examples. However, the present invention is not limited to these examples.

### Reference Example 1:

### Culture of cultured mammalian cells (derived from lymphoma)

Cultured mammalian cells L5178Y-S (2.1 × 10⁶ cells, obtained from the Cancer Cell Repository, Institute of Development, Aging and Cancer,Tohoku University)were cultured in a culture flask (2100 cm²) containing a RPMI1640 medium (manufactured by Invitrogen) supplemented with 10% fetus bovine serum (manufactured by Invitrogen) at 37°C in 5% CO₂ atmosphere for 70 hr. As a result, the cell count reached 4.2 × 10⁸ and wet weight 0.57 g.

### Example 1:

### Preparation of cultured mammalian cell (derived from lymphoma) extract liquid

First, the cultured mammalian cells cultured in Reference Example 1 were collected by centrifugation (700×g, 10 min), and were then washed (by centrifugation at 700×g for 10 min) 3 times with a wash solution having the following composition.

**(Composition of wash solution)**

| | |
|---|---|
| 50 mM | HEPES-KOH (pH 7.4) |
| 100 mM | potassium acetate |
| 2 mM | magnesium acetate |
| 2 mM | DTT |

Then, the cultured mammalian cells were suspended in a solution for extraction (cooled at 4°C) prepared by adding 0.57 mL of 0.5 mM PMSF and 20% glycerol to the wash solution having the above-mentioned composition.

The suspension was rapidly (within 2 sec) frozen in liquid nitrogen. After freezing sufficiently, the suspension was thawed in a water bath at about 10°C. After complete thawing, the suspension was subjected to centrifugation ("himacCR20B3", manufactured by Hitachi Koki) at 30,000xg, 4°C for 10 min to remove cell debris, and supernatant was recovered. The recovered supernatant was further subjected to centrifugation (4°C, 30,0000×g, 30 min) to recover supernatant, and the recovered supernatant was used as a culturedmammalian cell extract liquid.

### Reference Example 2:

### Preparation of exogenous mRNA

### (1) Construction of Vector DNA

Using pGEM-luc vector (5 ng, manufactured by Promega) as a template, a primer (Luc T7-F3-Kpn) having a base sequence depicted in SEQ ID; No 1, a primer (Luc T7-R4-Kpn) having a base sequence depicted in SEQ ID ; No 2, and KOD plus (manufactured by TOYOBO Co.), PCR was performed at 97°C, 15 sec, 55°C, 30 sec and 68°C, 120 sec, for 30 cycles. DNA fragment was purified by ethanol precipitation, and digested with KpnI. Separately, pT_{N}T Vector (manufactured by Promega) was digested with KpnI. These reaction liquids were separated by agarose gel electrophoresis, and using a Gen Elute Gel Purification Kit (manufactured by SIGMA), DNA fragment was purified. Using Ligation High (manufactured by TOYOBO Co.), these DNA fragments were ligated and *Escherichia coli* DH5α (manufactured by TOYOBO Co.) was transformed. Plasmid DNA was prepared from the transformed *Escherichia coli* by alkali-SDS methods, and subjected to a sequencing reaction (96°C 10 sec, 50°C 5 sec, 60°C 4 min, 30 cycles) using primer (T7 promoter) having a base sequence depicted in SEQ ID; No 3 and Big Dye Terminator Cycle Sequencing FS (manufactured by Applied Biosystems). This reaction liquid was applied to ABI PRISM 310 Genetic Analyzer (manufactured by Applied Biosystems), and the base sequence was analyzed. A plasmid having a start codon of luciferase gene inserted into the downstream of pT_{N}T Vector-derived 5'-β-globin leader sequence was named as pT_{N}T-Luc.

### (2) In Vitro Transcription Reaction

pT_{N}T-Luc prepared in the above-mentioned (1) was digested with BamHI, and purified by phenol-chloroform extraction and ethanol precipitation. By using 1 µg of the resultant DNA segment as a template, mRNA was synthesized using RiboMax Large Scale RNA Production System-T7 (manufactured by Promega) on a 20 µL scale at 37°C for 4 hr.

### (3) Purification of Exogenous mRNA

After the completion of the transcription reaction, 1 U RQ1 RNase free DNase (manufactured by Promega) was added to 20 µL of the reaction liquid. The mixture was incubated at 37°C for 15 min and the template DNA was digested. Protein was removed by phenol-chloroform extraction, and potassium acetate was added to the final concentration of 0.3 M to perform ethanol precipitation. The obtained precipitate was dissolved in 100 µL of distilled water and applied to Nick Column (manufactured by Amersham Biosciences) and eluted with distilled water (400 µL). The eluted fraction was recovered, potassium acetate was added to the final concentration of 0.3 M, and ethanol precipitation was conducted. For quantification of the synthesized mRNA, absorbance at 260 nm was measured.

### Experimental Example 1:

### Cell-free protein synthesis using cultured mammalian cell (derived from lymphoma) extract liquid

Using the cultured mammalian cell extract liquid prepared in Example 1 and exogenous mRNA synthesized in Reference Example 2, a reaction liquid having the following composition was prepared and protein synthesis in a cell-free system was performed.

**[Composition of reaction liquid]**

| | |
|---|---|
| 50 (v/v)% | cultured mammalian cell extract liquid |
| 25 mM | HEPES-KOH (pH 7.4) |
| 150 mM | potassium acetate |
| 1 mM | magnesium acetate |
| 1 mM | DTT |
| 0.5 mM | ATP |
| 0.1 mM | GTP |
| 20 mM | creatine phosphate |
| 200 µg/mL | creatine kinase |
| 0.25 mM | spermidine |
| 40 µM | amino acid (20 kinds) |
| 1 U/µL | RNase inhibitor (derived from human placenta) |
| 200 µg/mL | tRNA (derived from yeast) |
| 240 µg/mL | exogenous mRNA |
| 10 (v/v)% | glycerol |

ATP (manufactured by SIGMA), GTP (manufactured by SIGMA), amino acid (20 kinds, manufactured by SIGMA), RNase inhibitor (manufactured by TAKARA SHUZO Co.), and tRNA (manufactured by Roche Diagnostics) were respectively used. As a reaction device, low temperature dry block MG-1000 was used. The amount of the reaction liquid was 25 µL, the reaction temperature was 20°C, a sample was taken for each reaction time and the amount of synthesized luciferase was measured. The synthesized luciferase was quantified using a luciferase assay kit (E-1500, manufactured by Promega). The reaction liquid (2.5 µL) was added to a luciferase assay reagent (50 µL) and luminescence by luciferase was measured using a luminometer (Tuner Designs TD-20/20, manufactured by Promega).

The cell-free protein synthesis reaction continued for 1 hr after the start of the reaction, and as a result about 0.5 µg/mL of luciferase was synthesized.

### Comparative Example 1

Cultured mammalian cells L5178Y-S cultured in the same manner as in Reference Example 1 were washed with the same wash solution as used in Example 1 , and then were suspended in the same solution for extraction cooled at 4°C as used in Example 1. Thereafter, the same manner as in Example 1 was carried out except that the suspension was vigorously stirred for 5 min for extraction. Cell-free protein synthesis reaction was carried out using the mRNA prepared in Reference Example 2 and a reaction liquid having the same composition as the reaction liquid of Experimental Example 1.

As a result, the amount of synthesized luciferase was 10 ng/mL, which was about 1/50 of Experimental Example 1.

### Reference Example 3:

### Culture of cultured mammalian cells (CHO)

4.9 × 10⁵ cells/mL (cell concentration) of Chinese hamster ovary cells CHO K1-SFM (obtained from the Cancer Cell Repository, Institute of Development, Aging and Cancer, Tohoku University) were cultured in an Erlenmeyer flask (500 mL) containing 200 mL of a CHO SERUM-FREE MEDIUM (manufactured by SIGMA) at 130 rpm, 37°C in 5% CO₂ atmosphere for 120 hr. As a result, the cell concentration reached 8.8 × 10⁶ cells/mL and wet weight 3.2 g.

### Example 2:

### Preparation of cultured mammalian cell (CHO) extract liquid

First, the cultured mammalian cells cultured in Reference Example 3 were collected by centrifugation (700xg, 10 min), and were then washed (by centrifugation at 700xg for 10 min) 3 times with a buffer solution for washing having the following composition.

**(Composition of buffer solution for washing)**

| | |
|---|---|
| 40 mM | HEPES-KOH (pH 7.9) |
| 100 mM | potassium acetate |
| 2 mM | magnesium acetate |
| 2 mM | calcium chloride |
| 1 mM | DTT |

Then, the weight of the washed cells was measured, and 0. 8 mL of a buffer solution for extraction having the following composition was added per gram of the cells to obtain a cell suspension.

**(Composition of solution for extraction)**

| | |
|---|---|
| 40 mM | HEPES-KOH (pH 7.9) |
| 100 mM | potassium acetate |
| 2 mM | magnesium acetate |
| 2 mM | calcium chloride |
| 20%(v/v) | glycerol |
| 1 mM | DTT |

The cell suspension was rapidly frozen in liquid nitrogen. After freezing sufficiently, the suspension was thawed in an ice bath at about 4°C. After complete thawing, the suspension was subjected to centrifugation ("himac CR20B3" , manufactured by Hitachi Koki) at 30000xg, 4°C for 10 min to remove cell debris, and supernatant S1 was recovered. The recovered supernatant S1 was further subjected to centrifugation (4°C, 30000×g, 30 min) to recover supernatant S2. The supernatant S2 was applied to PD-10 (manufactured by Amersham Biosciences) equilibrated with a buffer solution for desalting having the following composition. After the application, the supernatant S2 was eluted with a buffer for desalting, and fractions having absorbance at 280 nm of not less than 30 were recovered using a spectrophotometer ("Ultrospec 3300 pro", manufactured by Amersham Biosciences) and used as a cultured mammalian cell (CHO) extract liquid.

**(Composition of buffer solution for desalting)**

| | |
|---|---|
| 40 mM | HEPES-KOH (pH 7.9) |
| 100 mM | potassium acetate |
| 2 mM | magnesium acetate |
| 1 mM | DTT |

### Experimental Example 2:

### Cell-free protein synthesis using cultured mammalian cell (CHO) extract liquid

The cultured mammalian cell (CHO) extract liquid prepared in Example 2 was mixed with components other than exogenous mRNA of a reaction liquid having the following composition, and the mixture was incubated at 25°C for 30 min. Then, the exogenous mRNA synthesized in Reference Example 2 was added to the mixture to synthesize a protein in a cell-free system.

**(Composition of reaction liquid)**

| | |
|---|---|
| 50%(v/v) | cultured mammalian cell extract liquid |
| 50 mM | HEPES-KOH (pH 7.9) |
| 175 mM | potassium acetate |
| 1 mM | magnesium acetate |
| 0.5 mM | calcium chloride |
| 2 mM | DTT |
| 0.5 mM | ATP |
| 0.25 mM | GTP |
| 30 mM | creatine phosphate |
| 200 µg/mL | creatine kinase |
| 80 µM | amino acid (20 kinds) |
| 160 µg/mL | mRNA (coding for the luciferase gene) |

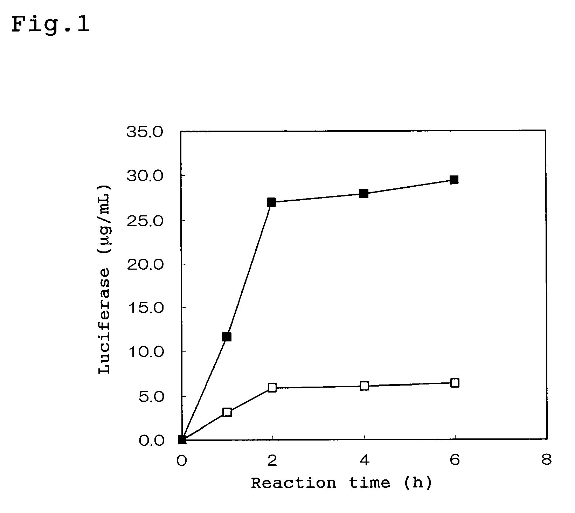

ATP (manufactured by SIGMA), GTP (manufactured by SIGMA), and amino acid (20 kinds, manufactured by SIGMA) were used. As a reaction device, a low temperature dry block MG-1000 was used. The amount of the reaction liquid was 25 µL, the reaction temperature was 25°C, and a sample was taken for each reaction time to measure the amount of synthesized luciferase. The synthesized luciferase was quantified using a luciferase assay kid (E-1500, manufactured by Promega). 2.5 µL of the reaction liquid was added to 50 µL of a luciferase assay reagent, and luminescence by luciferase was measured using a luminometer (Tuner Designs TD-20/20, manufactured by Promega). As a result, the protein synthesis reaction continued for 2 hr from the start of the reaction, and about 27 µg/mL of protein was synthesized (■ in Fig. 1), which was significantly larger as compared to a case where incubation (25°C, 30 min) was not carried out prior to conducting protein synthesis (□ in Fig. 1).

As is apparent from the above description, according to the present invention, it is possible to provide a method for preparing a culturedmammalian cell extract liquid for cell-free protein synthesis which is easy to prepare and affords synthesis of a larger amount of protein than before, the cultured mammalian cell extract liquid, and a method for cell-free protein synthesis using the cultured mammalian cell.

In free text of Sequence Listing (Description of Artificial Sequence), SEQ ID; No 1 is Primer Luc T7-F3-Kpn, SEQ ID; No 2 is Primer Luc T7-R4-Kpn, and SEQ ID; No 3 is Primer T7 promoter.

## Claims

1. A method for preparing a cultured mammalian cell extract liquid, comprising at least the step of rapidly freezing a cultured mammalian cell suspended in a solution for extraction.

2. The preparation method according to claim 1, further comprising the step of thawing the cultured mammalian cell after rapid freezing, and subjecting the cell to centrifugation.

3. The preparation method according to claim 1 or 2, wherein the cultured mammalian cell is rapidly frozen with liquid nitrogen.

4. The preparation method according to claim 2 or 3, wherein the cultured mammalian cell rapidly frozen is thawed in a water bath or ice-water bath at -10°C - 20°C.

5. The preparation method according to any one of claims 1 to 4, wherein the solution for extraction contains a protease inhibitor.

6. The preparation method according to any one of claims 1 to 5, wherein the cultured mammalian cell is a cultured cell derived from lymphoma.

7. The preparation method according to any one of claims 1 to 5, wherein the cultured mammalian cell is a cultured cell derived from a gonad.

8. The preparation method according to claim 7, wherein the cultured mammalian cell is a cultured cell derived from Chinese hamster ovary (CHO).

9. The preparation method according to claim 8, wherein the Chinese hamster ovary (CHO) is derived from CHO K1-SFM.

10. A cultured mammalian cell extract liquid, which is prepared by the method according to any one of claims 1 to 9.

11. A method for cell-free protein synthesis, comprising use of the cultured mammalian cell extract liquid according to claim 10.

12. The method for cell-free protein synthesis according to claim 11, comprising the steps of;
effecting incubation of a reaction liquid for cell-free protein synthesis containing components other than an exogenous mRNA, and then
adding an exogenous mRNA into the reaction liquid to conduct synthetic reaction.

13. The method for cell-free protein synthesis according to claim 12, wherein the incubation is carried out at 0°C to 50°C .
